# EUROPEAN PATENT APPLICATION

(11) **EP 4 545 932 A1**
(43) Date of publication of application: **30.04.2025**
(21) Application number: 23826798.3
(22) Date of filing: 26.04.2023
(51) Int. Cl.: G01N 7/04, B01D 53/62

(54) **CONCENTRATION MEASUREMENT METHOD, AND RECOVERY METHOD AND RECOVERY DEVICE FOR CARBON DIOXIDE**

(30) Priority: 24.06.2022 JP 2022101746
(71) Applicant: Niterra Co., Ltd., Nagoya-shi, Aichi 461-0005 (JP)
(72) Inventor: GOTO Yuta, Nagoya-shi, Aichi 461-0005 (JP)
(74) Representative: Grünecker Patent- und Rechtsanwälte PartG mbB
(86) International application number: PCT/JP2023/016373
(87) International publication number: WO 2023/248609

(57) **Abstract**

The present invention provides a concentration measurement method that can accurately determine the carbon dioxide concentration in a desorption gas using a general-purpose sensor, and a recovery method and a recovery device (10) for carbon dioxide. Provided is a concentration measurement method for measuring the carbon dioxide concentration in desorption gas that includes carbon dioxide desorbed from an adsorbent which has adsorbed carbon dioxide contained in a gas, said method comprising: a first process for detecting the flow rate and the carbon dioxide concentration of a gas; a second process for detecting the flow rate and the carbon dioxide concentration of a residual gas after an adsorbent has adsorbed carbon dioxide from the gas; and a calculation process for determining the carbon dioxide concentration of a desorption gas from the result of the first process and the result of the second process.

## Description

### TECHNICAL FIELD

The present invention relates to a method for measuring carbon dioxide concentration (hereinafter may also be referred to as a "carbon dioxide concentration measurement method"), to a method for recovering carbon dioxide (hereinafter may also be referred to as a "carbon dioxide recovery method"), and to a device for recovering carbon dioxide (hereinafter may also be referred to as a "carbon dioxide recovery device").

### BACKGROUND ART

According to a technique disclosed in Patent Literature 1 in relation to a carbon dioxide recovery device employing pressure swing adsorption, which is a technique of modifying the affinity of carbon dioxide to an adsorbent by pressure, the carbon dioxide concentration of a gas containing carbon dioxide desorbed (i.e., released) from the adsorbent (the gas being referred to as a "desorption gas") is detected, to thereby control a carbon dioxide recovery device.

### CITATION LIST

### PATENT LITERATURES

Patent Literature 1: JP2016-40025A

### SUMMARY OF INVENTION

### TECHNICAL PROBLEM

In the related art, difficulty is encountered in accurately detecting the carbon dioxide concentration of a desorption gas, since the flow rate of a desorption gas varies considerably, which is problematic. In addition, carbon dioxide is concentrated in a desorption gas. Thus, a particular sensor that can detect high-concentration carbon dioxide is required, which is also problematic.

The present invention has been established so as to solve the above problems. Thus, an object of the present invention is to provide a method for accurately determining the carbon dioxide concentration of a desorption gas by means of a general-purpose sensor. Other objects are to provide a carbon dioxide recovery method and a carbon dioxide recovery device.

### SOLUTION TO PROBLEM

In order to attain the aforementioned objects, a first embodiment of the present invention is directed to a concentration measurement method for measuring a carbon dioxide concentration of a desorption gas containing carbon dioxide desorbed from an adsorbent which has adsorbed carbon dioxide contained in a target gas. The method includes a first processing of detecting a flow rate and a carbon dioxide concentration of the target gas; a second processing of detecting a flow rate and a carbon dioxide concentration of a residual gas resulting from adsorption of carbon dioxide contained in the target gas by the adsorbent; and a calculation processing of determining a carbon dioxide concentration of the desorption gas on the basis of the results of the first processing and the second processing.

A second embodiment of the invention is directed to a carbon dioxide recovery method, the method including an adsorption processing of causing carbon dioxide contained in a target gas to be adsorbed by an adsorbent; and a desorption processing of desorbing carbon dioxide from the adsorbent, to thereby yield a desorption gas containing carbon dioxide. In the recovery method, any one or more physical properties from among a time of the adsorption processing, a flow rate of a residual gas provided through adsorption of carbon dioxide contained in the target gas by the adsorbent in the adsorption processing, a time of the desorption processing, and a flow rate of the desorption gas are controlled on the basis of the carbon dioxide concentration of the desorption gas obtained in the first embodiment.

A third embodiment of the invention further includes, in addition to the second embodiment, a purge processing of removing, by means of the residual gas, carbon dioxide present around the adsorbent from which carbon dioxide has been desorbed. In the third embodiment, any one or more physical properties from among a time of the purge processing and a flow rate of the residual gas employed in the purge processing are controlled on the basis of the carbon dioxide concentration of the desorption gas.

A fourth embodiment of the invention is directed to a carbon dioxide recovery method, the method including an adsorption processing of causing carbon dioxide contained in a target gas to be adsorbed by an adsorbent; and a desorption processing of desorbing carbon dioxide from the adsorbent, to thereby yield a desorption gas containing carbon dioxide. The recovery method further includes a mixing process of controlling the amount of the desorption gas added to the target gas on the basis of the carbon dioxide concentration of the desorption gas obtained in the first embodiment. In the adsorption processing, a gas mixture formed by mixing the target gas with the desorption gas is brought into contact with the adsorbent.

A fifth embodiment of the invention is directed to a carbon dioxide recovery device which is adapted to cause carbon dioxide contained in a target gas to be adsorbed by an adsorbent, to desorb carbon dioxide from the adsorbent, and to yield a desorption gas containing carbon dioxide. The recovery device includes a first measurement unit that can detect a flow rate and a carbon dioxide concentration of the target gas; a second measurement unit that can detect a flow rate and a carbon dioxide concentration of a residual gas resulting from adsorption of carbon dioxide contained in the target gas by the adsorbent; and a calculation unit that can determine a carbon dioxide concentration of the desorption gas on the basis of the measurement results obtained in the first measurement unit and the second measurement unit.

A sixth embodiment of the invention further includes, in addition to the fifth embodiment, a control unit that can control, on the basis of the carbon dioxide concentration of the desorption gas, any one or more physical properties from among a time of an adsorption processing of causing carbon dioxide contained in a target gas to be adsorbed by the adsorbent, a flow rate of a residual gas resulting from adsorption of carbon dioxide contained in the target gas by the adsorbent in the adsorption processing, a time of a desorption processing of desorbing carbon dioxide from the adsorbent, to thereby yield a desorption gas containing carbon dioxide, and a flow rate of the desorption gas.

The recovery device of a seventh embodiment of the invention further includes, in addition to the fifth or sixth embodiment, a purge unit adapted to conduct a purge processing of removing, by means of the residual gas, carbon dioxide present around the adsorbent from which carbon dioxide has been desorbed. The purge unit can control any one or more physical properties from among a time of the purge processing and a flow rate of a residual gas in the purge processing on the basis of the carbon dioxide concentration of the desorption gas.

The recovery device of an eighth embodiment of the invention further includes, in addition to any of the fifth to seventh embodiments, a mixing unit adapted to conduct a mixing process of controlling the amount of the desorption gas mixed with the target gas on the basis of the carbon dioxide concentration of the desorption gas. In the mixing unit, a gas mixture formed by mixing the target gas with the desorption gas is brought into contact with the adsorbent.

### ADVANTAGEOUS EFFECT OF INVENTION

According to the concentration measurement method of the present invention, the carbon dioxide concentration of the target gas and that of the residual gas are lower than the carbon dioxide concentration of the desorption gas. Thus, the carbon dioxide concentration of the target gas and that of the residual gas can be detected by means of a general-purpose sensor. Also, the variation in the flow rates of the target gas and the residual gas is smaller, as compared with the variation in flow rate of the desorption gas. Thus, the carbon dioxide concentration and the flow rate can be accurately detected through the first processing and the second processing. As a result, the carbon dioxide concentration of the desorption gas can be accurately determined through the calculation processing.

According to the carbon dioxide recovery method of the present invention, any one or more physical properties from among the time of the adsorption processing, the flow rate of a residual gas resulting from adsorption of carbon dioxide contained in the target gas by the adsorbent in the adsorption processing, the time of the desorption processing, and the flow rate of the desorption gas are controlled on the basis of the carbon dioxide concentration of the desorption gas obtained through the concentration measurement method. Since the carbon dioxide concentration of the desorption gas can be accurately determined, the flow rate and the processing time are controlled on the basis of an accurate carbon dioxide concentration, whereby the percent recovery of carbon dioxide can be enhanced.

According to the carbon dioxide recovery method of the present invention, the amount of the desorption gas added to the target gas in the mixing process is controlled on the basis of the carbon dioxide concentration of the desorption gas obtained through the concentration measurement method, and a gas mixture formed by mixing the target gas with the desorption gas is brought into contact with the adsorbent in the adsorption processing. Since the carbon dioxide concentration of the desorption gas is accurately determined, the amount of the desorption gas contained in the gas mixture is controlled on the basis of an accurate carbon dioxide concentration, and the gas mixture is brought into contact with the adsorbent, whereby the percent recovery of carbon dioxide can be enhanced.

According to the recovery device of the present invention, the carbon dioxide concentration and the flow rate can be accurately detected in the first measurement unit and the second measurement unit. Thus, the carbon dioxide concentration of the desorption gas can be accurately determined in the calculation unit.

### BRIEF DESCRIPTION OF DRAWINGS

[FIG. 1] Piping system diagram of a recovery device in one embodiment.
[FIG. 2] Block diagram of a control device.

### DESCRIPTION OF EMBODIMENT

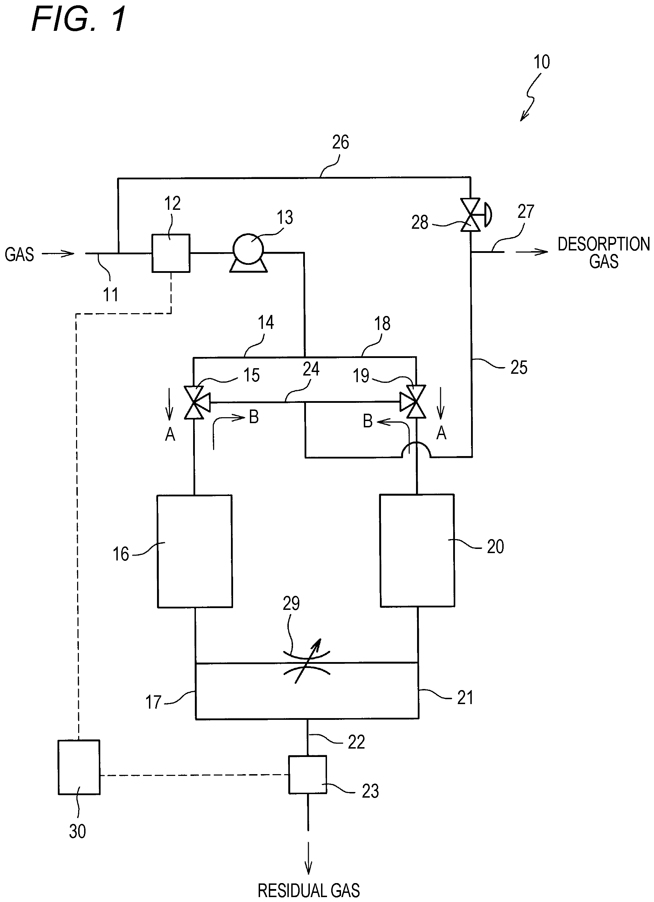

In reference to the attached drawings, a preferred embodiment of the present invention will now be described. FIG. 1 is a piping system diagram of a recovery device 10 in one embodiment. The recovery device 10 is a device for recovering carbon dioxide contained in gas through employment of pressure swing adsorption. Examples of the gas include exhaust gases discharged from, for example, power plant, plant, waste treatment facility, natural gas field, and oil field.

The recovery device 10 has an intake pipe 11 through which a target gas is fed; intake pipes 14, 18 which are branched from the intake pipe 11 and connected to two adsorption columns 16, 20, respectively; exhaust pipes 17, 21 which are connected to the adsorption columns 16, 20, respectively; and an exhaust pipe 22 which is connected to the exhaust pipes 17, 21 to merge the exhaust pipes 17, 21. To the exhaust pipe 22, a second measurement unit 23 is disposed.

In the intake pipe 11, there are disposed a first measurement unit 12 and a pressurizing device 13, from the upstream side The first measurement unit 12 includes a flow meter that can detect the flow rate of the gas flowing through the intake pipe 11, and a concentration meter that can detect the carbon oxide concentration of the gas flowing through the intake pipe 11. Examples of the concentration meter include a general-purpose sensor employing a relatively inexpensive non-dispersive infrared (NDIR) mode. In the first measurement unit 12, the flow meter and the concentration meter may be integrated.

The pressurizing device 13 is a device for pressurizing a gas flowing through the intake pipe 11 so as to appropriately tune the pressure of the adsorption columns 16, 20 to an appropriate level, and examples thereof include a compressor and a blower. Each of the adsorption columns 16, 20 accommodates an adsorbent. Examples of the adsorbent include activated carbon, zeolite, mesoporous silica, metal organic framework (MOF), and porous coordination polymer (PCP).

The intake pipe 11 is branched to the intake pipes 14, 18 on the downstream side of the pressurizing device 13. The intake pipes 14, 18 are equipped with three-way valves (3-port valves) 15, 19, respectively. In the three-way valve 15, two ports are connected to the intake pipe 14, and the remaining one port is connected to a recovering pipe 24. The three-way valve 15 is a solenoid operated valve and switches, by an input electric signal, to a flow path A through which a gas is transferred from the intake pipe 11 to the adsorption column 16 via the intake pipe 14 (denoted by arrow A), and a flow path B through which a gas is transferred from the adsorption column 16 to the recovering pipe 24 (denoted by arrow B).

In the three-way valve 19, two ports are connected to the intake pipe 18, and the remaining one port is connected to a recovering pipe 24. The three-way valve 19 is a solenoid operated valve and switches, by an input electric signal, to a flow path A through which a gas is transferred from the intake pipe 11 to the adsorption column 20 via the intake pipe 18 (denoted by arrow A), and a flow path B through which a gas is transferred from the adsorption column 20 to the recovering pipe 24 (denoted by arrow B).

A recovering pipe 25 is connected to the midpoint of the recovering pipe 24, which is connecting the three-way valves 15, 19 to each other. To an end of the recovering pipe 25, a first end of a mixing pipe 26 is connected. A second end of the mixing pipe 26 is connected to the intake pipe 11 on the upstream side of the first measurement unit 12. The recovering pipe 25 is branched to a recovering pipe 27 from the point where the first end of the mixing pipe 26 is connected.

In the mixing pipe 26, a flow control valve 28 is disposed. When the flow control valve 28 is closed, the entirety of the gas flowing through the recovering pipe 25 is transferred to the recovering pipe 27. By modifying the opening of the flow control valve 28, the volume of a portion of the gas flowing through the recovering pipe 25 to the mixing pipe 26 can be controlled.

The second measurement unit 23 includes a flow meter that can detect the flow rate of the gas flowing through the exhaust pipe 22, and a concentration meter that can detect the carbon oxide concentration of the gas flowing through the exhaust pipe 22. Examples of the concentration meter include a general-purpose sensor employing a relatively inexpensive non-dispersive infrared absorption (NDIR) mode. In the second measurement unit 23, the flow meter and the concentration meter may be integrated.

Between the exhaust pipe 17 and the exhaust pipe 21, a variable throttle valve 29 is disposed to connect the pipes. The exhaust pipes 17, 21, 22 and the variable throttle valve 29 are arranged such that the ratio in flow rate of the intake pipe 14 or the intake pipe 18 to the exhaust pipe 22 is adjusted to fall within a range, for example, 10:2 to 10:4. The variable throttle valve 29 has a needle valve form, and can seamlessly control the flow rate between the exhaust pipe 17 and the exhaust pipe 21 by modifying the opening. Generally, the opening of the variable throttle valve 29 is set to a constant value. The recovery device 10 has a control device 30.

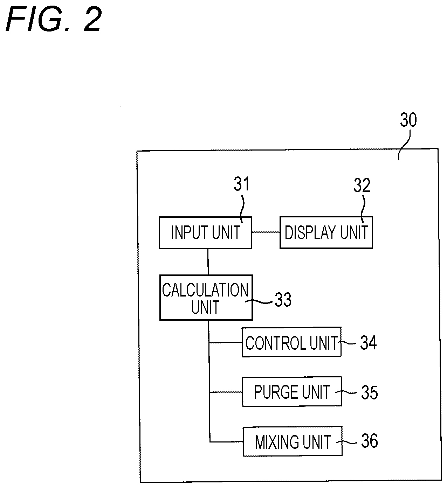

FIG. 2 is a block diagram of the control device 30. The control device 30 has an input unit 31 to which the detection results obtained by the first measurement unit 12 and the second measurement unit 23 are input; a display unit 32 that can display the input/output results on a liquid crystal panel or the like; a calculation unit 33 for calculating the carbon dioxide concentration of the desorption gas on the basis of the detection results obtained by the first measurement unit 12 and the second measurement unit 23; a control unit 34 and a purge unit 35 for switching the three-way valves 15, 19 on the basis of the output of the calculation unit 33; and a mixing unit 36 for modulating the opening of the flow control valve 28. The control unit 34 and the purge unit 35 may also modulate the opening of the variable throttle valve 29.

The control device 30 has a CPU, a ROM, and a RAM (all being not illustrated). In the ROM, programs for conducting calculating carbon dioxide concentration, switching of the three-way valves 15, 19, modulating the opening of the flow control valve 28, etc. are stored. The CPU conducts relevant operations in the calculation unit 33, the control unit 34, and the purge unit 35, on the basis of the programs stored in the ROM.

An example of the carbon dioxide recovery method employing the recovery device 10 will be described. In the example, recovering carbon dioxide which has been adsorbed by the adsorption column 20 during adsorption of carbon dioxide by the adsorption column 16 is carried out; and then recovering carbon dioxide which has been adsorbed by the adsorption column 16 during adsorption of carbon dioxide by the adsorption column 20 is carried out. Firstly, the control unit 34 and the purge unit 35 set the flow path A to the three-way valve 15, and the flow path B to the three-way valve 19.

When a gas (exhaust gas) is supplied to the intake pipe 11 at a specific flow rate to activate the pressurizing device 13, the gas is transferred to the adsorption column 16 through the intake pipe 14, whereby the pressure of the adsorption column 16 increases. As a result, carbon dioxide contained in the gas is adsorbed by the adsorbent present in the adsorption column 16 (adsorption processing). A residual gas resulting from adsorption of carbon dioxide in the gas by the adsorbent (i.e., a gas having a carbon dioxide partial pressure lower than that of the exhaust gas) is discharged from the adsorption column 16 to the outside of the recovery device 10 through the exhaust pipes 17, 22. A part of the residual gas enters the adsorption column 20 through the variable throttle valve 29.

Meanwhile, carbon dioxide adsorbed by the adsorbent present in the adsorption column 20 connected to the three-way valve 19 disposed in the flow path B is desorbed (i.e., desorption processing). The desorption gas containing carbon dioxide desorbed from the adsorbent present in the adsorption column 20 is transferred through the recovering pipes 24, 25 and recovered through the recovering pipe 27. The residual gas which has been discharged from the adsorption column 16 and has entered the adsorption column 20 through the variable throttle valve 29 relatively reduces the carbon dioxide partial pressure in the adsorption column 20, to thereby promote desorption of carbon oxide from the adsorbent. As a result, the desorption gas present in the adsorption column 20 is extruded to the recovering pipe 24, to thereby purify the adsorption column 20 (purge processing). The desorption processing and the purge processing in the adsorption column 20 are performed in parallel with the adsorption processing in the adsorption column 16.

The adsorption performance of the adsorbent in the adsorption column 16 decreases as carbon dioxide is adsorbed by the adsorbent. Thus, the carbon dioxide concentration of the desorption gas discharged from the adsorption column 16 increases over time. In contrast, the carbon dioxide concentration of the residual gas discharged from the adsorption column 20 gradually decreases over time. Thus, after passage of a certain period of time, the control unit 34 and the purge unit 35 switch the three-way valves 15, 19, to thereby set the three-way valve 15 to the flow path B, and then set the three-way valve 19 to the flow path A. By setting the three-way valve 15 to the flow path B, the pressure of the adsorption column 16 is adjusted to atmospheric pressure.

Once the three-way valve 19 is set to the flow path A, the gas pressurized by the pressurizing device 13 enters the adsorption column 20 through the intake pipe 18, whereby the pressure of the adsorption column 20 increases. Thus, carbon oxide contained in the gas is adsorbed by the adsorbent present in the adsorption column 20 (adsorption processing). The residual gas resulting in adsorption of carbon dioxide contained in the gas by the adsorbent is discharged to the outside of the recovery device 10 from the adsorption column 20 through the exhaust pipes 21, 22. A part of the residual gas discharged to the outside of the recovery device 10 enters the adsorption column 16 through the variable throttle valve 29.

Meanwhile, carbon dioxide adsorbed by the adsorbent present in the adsorption column 16 connected to the three-way valve 15 disposed in the flow path B is desorbed (i.e., desorption processing). The desorption gas containing carbon dioxide desorbed from the adsorbent present in the adsorption column 16 is transferred through the recovering pipes 24, 25 and recovered through the recovering pipe 27. The residual gas which has been discharged from the adsorption column 16 and has entered the adsorption column 16 through the variable throttle valve 29 relatively reduces the carbon dioxide partial pressure in the adsorption column 20, to thereby promote desorption of carbon oxide from the adsorbent. As a result, the desorption gas present in the adsorption column 16 is extruded to the recovering pipe 24, to thereby purify the adsorption column 16 (purge processing). The desorption processing and the purge processing in the adsorption column 16 are performed in parallel with the adsorption processing in the adsorption column 20.

The control unit 34 and the purge unit 35 repeatedly switch the three-way valves 15, 19, to thereby conduct the adsorption processing alternatingly in the adsorption columns 16, 20. As a result, a desorption gas containing concentrated carbon dioxide is recovered through the recovering pipe 27. The thus-recovered carbon dioxide may produce, for example, methane through reaction with hydrogen. Alternatively, carbon dioxide and hydrogen serving as raw materials may be converted to an intermediate such as syngas, methanol, or ethanol, whereby fuel such as light oil and gasoline, BTX (benzene, toluene, and xylene), DME (dimethyl ether), butadiene, and chemical products can be produced from the intermediate.

The first measurement unit 12 detects the flow rate F1 and the carbon dioxide concentration C1 of the gas flowing through the intake pipe 11 (first processing). The second measurement unit 23 detects the flow rate F2 and the carbon dioxide concentration C2 of the residual gas flowing through the exhaust pipe 22 (second processing). The flow rate (by mass) of carbon dioxide contained in the gas flowing the intake pipe 11 is represented by F1×C1. The flow rate (by mass) of carbon dioxide contained in the residual gas flowing the exhaust pipe 22 is represented by F2×C2.

Since the gas flowing through the intake pipe 11 is branched into a residual gas flowing through the exhaust pipe 22 and a desorption gas flowing through the recovering pipe 25, the flow rate F3 of the desorption gas flowing through the recovering pipe 25 corresponds to a flow rate F1-F2, obtained by subtracting the flow rate F2 of the residual gas from the flow rate F1 of the gas. Also, since carbon dioxide contained in the gas is divided into carbon dioxide contained in the residual gas and carbon dioxide contained in the desorption gas, the flow rate (by mass) of carbon dioxide contained in the desorption gas corresponds to F1×C1 - F2×C2, obtained by subtracting the flow rate (by mass) F2×C2 of carbon dioxide contained in the residual gas from the flow rate (by mass) F1×C1 of carbon dioxide contained in the gas. Accordingly, the carbon dioxide concentration C3 of the desorption gas flowing through the recovering pipe 25 corresponds to C3=(F1×C1 - F2×C2)/(F1 - F2), obtained by dividing the flow rate (by mass) of carbon dioxide contained in the desorption gas F1×C1 - F2×C2 by the flow rate F3 (=F1 - F2) of the desorption gas (calculation processing). Thus, the calculation unit 33 outputs the carbon dioxide concentration C3.

When the carbon dioxide concentration C3 of the desorption gas is below a target level, the mixing unit 36 increases the opening of the flow control valve 28, whereby the flow rate of the desorption gas transferred from the recovering pipe 25 to mixing pipe 26 can be increased (mixing process). The desorption gas transferred from the mixing pipe 26 to the intake pipe 11 intermingles with a gas flowing through the intake pipe 11. As a result, recovery of the desorption gas having a carbon dioxide concentration C3 below a target level can be avoided. A gas mixture of the above gas and the desorption gas enters the adsorption column 16 or the adsorption column 20 via the pressurizing device 13, and carbon dioxide contained in the gas mixture is adsorbed by the adsorbent.

In contrast, when the carbon dioxide concentration C3 of the desorption gas is equal to or higher than a target level, the mixing unit 36 decreases the opening of the flow control valve 28, whereby the flow rate of the desorption gas transferred from the recovering pipe 25 to the mixing pipe 26 is reduced, to thereby increase the flow rate the desorption gas flowing through the recovering pipe 27 (mixing process). In this case, the flow control valve 28 may be completely closed. Since only the desorption gas containing carbon dioxide having a high carbon dioxide concentration equal to or higher than a target level is discharged out to the recovery device 10 via the recovering pipe 27, percent recovery of carbon dioxide can be enhanced. Needless to say, in the mixing process, the opening of the flow control valve 28 can be continuously modulated in response to the carbon dioxide concentration C3 of the desorption gas.

Since the amount of the gas (exhaust gas) fed to the intake pipe 11 at a specific flow rate is much more than the amount of the desorption gas fed from the mixing pipe 26 to the intake pipe 11, the flow rate of the gas (including gas mixture) passing through the first measurement unit 12 is substantially constant. In addition, the flow rate of the residual gas passing through the second measurement unit 23 is adjusted to a substantially constant level by means of the variable throttle valve 29. When variation in flow rate of gas is large, accuracy of detection of a carbon dioxide concentration by means of a gas sensor is poor. However, when variation in flow rate of gas is small, the carbon dioxide concentration can be accurately detected. Thus, the carbon dioxide concentration C1 of the gas (including gas mixture) can be accurately detected by means of the first measurement unit 12, and the carbon dioxide concentration C2 of the residual gas can be accurately detected by means of the second measurement unit 23.

Further, the target gas is provided before concentration of carbon dioxide by means of the adsorption columns 16, 20, and the residual gas is provided after adsorption of carbon oxide contained in the target gas by the adsorbent. Therefore, the carbon dioxide concentration C1 of the target gas and the carbon dioxide concentration C2 of the residual gas are lower than carbon dioxide concentration C3 of the desorption gas in which carbon dioxide has been concentrated. In addition, the desorption gas having a carbon dioxide concentration C3 below a target level passes through the mixing pipe 26 and intermingles with the gas flowing through the intake pipe 11, no substantial rise in carbon dioxide concentration of the gas mixture is observed.

Thus, the carbon dioxide concentration of the residual gas and the target gas (containing gas mixture) can be detected by means of a general-purpose, relatively inexpensive sensor such as a sensor employing an NDIR mode, without employing a particular, expensive sensor that can detect high-concentration carbon dioxide. According to the recovery device 10, the carbon dioxide concentration C3 of the desorption gas can be more accurately determined through a calculation processing on the basis of the detection results obtained by the first measurement unit 12 and the second measurement unit 23 employed in a general-purpose sensor, without directly detecting the carbon dioxide concentration of the desorption gas. As a result, a desorption gas having a high carbon dioxide concentration can be recovered, while high percent recovery is maintained.

Hereinabove, the present invention has been described with reference to the embodiment. However, the present invention should not be limited to the aforementioned embodiment. Those skilled in the art can easily infer that various variations and modifications are possible so long as they are not deviated from the gist of the present invention.

For example, the aforementioned piping system of the recovery device 10 is merely an example and may be appropriately modified. In the aforementioned embodiment, the recovery device 10 has two adsorption columns 16, 20. However, the configuration is not necessarily limited thereto. Needless to say, 3 or more adsorption columns may be disposed, and an adsorption processing and a desorption processing may be sequentially performed in the 3 or more adsorption columns.

In the aforementioned embodiment, the recovery device 10 employing a PSA method been described. In the PSA method, a target gas is fed to the adsorption columns 16, 20 at a pressure corresponding to adsorption pressure, and desorption is evoked at generally atmospheric pressure. However, the device is not necessarily limited thereto. Needless to say, there may be employed a recovery device employing the VSA method or the PVSA method, in which adsorption pressure is achieved by rising the pressure of a target gas, and desorption is evoked under reduced pressure by means of a vacuum pump, equipped with the first measurement unit 12, the second measurement unit 23, and the calculation unit 33.

In the aforementioned embodiment, the variable throttle valve 29 is used to ligate the exhaust pipe 17 and the exhaust pipe 21. However, the configuration is not necessarily limited thereto. Alternatively, the variable throttle valve 29 may be changed to a plurality of orifices so as to juxtapose the exhaust pipe 17 and the exhaust pipe 21, and an open/close valve may be provided in each orifice. Similarly, in this case, the flow rate between the exhaust pipe 17 and the exhaust pipe 21 can be tuned by controlling the open/close valves. Needless to say, in order to omit tuning of the flow rate between the exhaust pipe 17 and the exhaust pipe 21, the exhaust pipe 17 and the exhaust pipe 21 may be ligated by means of an orifice having a fixed opening. Also, needless to say, a plurality of valves that can realize the function of three-way valves may be disposed instead of the three-way valves 15, 19.

In the aforementioned embodiment, after passage of a certain period of time, the control unit 34 switches the adsorption columns 16, 20, in which the adsorption processing is executed, through switching the three-way valves 15, 19. However, the operation mode is not necessarily limited thereto. Needless to say, instead of switching the adsorption columns 16, 20, in which the adsorption processing is executed, on the basis of the time of passage, the adsorption columns 16, 20, in which the adsorption processing is executed, may be switched on the basis of the carbon dioxide concentration C2 of the residual gas detected by the second measurement unit 23. Since the maximum amount of carbon dioxide adsorbed by the adsorbent is limited, the adsorption performance of the adsorbent decreases, as the adsorbent adsorbs more carbon dioxide, whereby the carbon dioxide concentration C2 of the residual gas increases. Thus, when the carbon dioxide concentration C2 exceeds a specific level, adsorption performance of the adsorbent can be constantly secured by switching the adsorption columns 16, 20, in which the adsorption processing is executed.

In the aforementioned embodiment, after passage of a certain period of time, the purge unit 35 switches the adsorption columns 16, 20, in which the desorption processing and the purge processing are executed, through switching the three-way valves 15, 19. However, the operation mode is not necessarily limited thereto. Needless to say, instead of switching the adsorption columns 16, 20, in which the desorption processing and the purge processing are executed, on the basis of the time of passage, the adsorption columns 16, 20, in which the desorption processing and the purge processing are executed, may be switched on the basis of the carbon dioxide concentration C3 of the desorption gas. Since the adsorbent desorbs carbon dioxide which has been adsorbed, the carbon dioxide concentration C3 of the desorption gas decreases in the progress of desorption of carbon dioxide. Thus, when the carbon dioxide concentration C3 decreases to a level below a target value, a desorption gas having a carbon dioxide concentration C3 equal to or higher than the target level can be consistently recovered by switching the adsorption columns 16, 20, in which the desorption processing and the purge processing are executed. As a result, percent recovery of carbon dioxide can be enhanced.

In the aforementioned embodiment, the desorption processing and the purge processing are executed in the recovery device 10, when the opening of the variable throttle valve 29 is fixed. However, the operation mode is not necessarily limited thereto. Needless to say, the control unit 34 and the purge unit 35 can modulate the opening of the variable throttle valve 29 on the basis of the carbon dioxide concentration C3 of the desorption gas, and can regulate the flow rate of the residual gas flowing through the exhaust pipe 22 and the flow rate of the desorption gas flowing through the recovering pipe 25.

For example, when the carbon dioxide concentration C3 of the desorption gas is equal to or higher than a target level in the desorption processing or the purge processing, the control unit 34 or the purge unit 35 may increase the opening of variable throttle valve 29. When the opening of the variable throttle valve 29 increases, the flow rate of the residual gas flowing through the exhaust pipe 22 decreases; and the flow rate of the residual gas transferred from the adsorption column 16 to the adsorption column 20, or the flow rate of the residual gas transferred from the adsorption column 20 to the adsorption column 16 increases. Further, the flow rate of the desorption gas flowing through the recovering pipe 25 increases. When the flow rate of the residual gas transferred from the adsorption column 16 to the adsorption column 20, or the flow rate of the residual gas transferred from the adsorption column 20 to the adsorption column 16 increases, the carbon dioxide partial pressure in the adsorption columns 16, 20 in which the desorption processing and the purge processing are executed, rapidly decreases in a relative manner, whereby the carbon dioxide desorption speed increases. As a result, percent recovery of carbon dioxide can be enhanced. Needless to say, in the desorption processing and the purge processing, the control unit 34 and the purge unit 35 can continuously modulate the opening of the variable throttle valve 29 in response to the carbon dioxide concentration C3 of the desorption gas.

In the aforementioned embodiment, a target gas (exhaust gas) is fed to the intake pipe 11 of the recovery device 10 at a specific flow rate. However, the operation mode is not necessarily limited thereto. Needless to say, the control unit 34 can modulate the flow rate of the gas fed to the intake pipe 11 on the basis of the carbon dioxide concentration C3 of the desorption gas, so long as the detection accuracy in the first measurement unit 12 is not impaired.

For example, when the carbon dioxide concentration C3 of the desorption gas exceeds a target level, the control unit 34 may increase the flow rate of the gas fed to the intake pipe 11. When the flow rate of the gas fed to the intake pipe 11 increases, the flow rate of the residual gas flowing through the exhaust pipe 22 increases, and the flow rate of the residual gas transferred from the adsorption column 16 to the adsorption column 20, or the flow rate of the residual gas transferred from the adsorption column 20 to the adsorption column 16 increases. Further, the flow rate of the desorption gas flowing through the recovering pipe 25 increases. As a result, percent recovery of carbon dioxide can be enhanced.

### REFERENCE SIGNS LIST

10 recovery device
12 first measurement unit
23 second measurement unit
33 calculation unit
34 control unit
35 purge unit
36 mixing unit

## Claims

1. A concentration measurement method for measuring a carbon dioxide concentration of a desorption gas containing carbon dioxide desorbed from an adsorbent which has adsorbed carbon dioxide contained in a target gas, the method comprising:
a first processing of detecting a flow rate and a carbon dioxide concentration of the target gas;
a second processing of detecting a flow rate and a carbon dioxide concentration of a residual gas resulting from adsorption of carbon dioxide contained in the target gas by the adsorbent; and
a calculation processing of determining a carbon dioxide concentration of the desorption gas on the basis of the results of the first processing and the second processing.

2. A carbon dioxide recovery method, the method comprising:
an adsorption processing of causing carbon dioxide contained in a target gas to be adsorbed by an adsorbent; and
a desorption processing of desorbing carbon dioxide from the adsorbent, to thereby yield a desorption gas containing carbon dioxide, wherein
any one or more physical properties from among a time of the adsorption processing, a flow rate of a residual gas resulting from adsorption of carbon dioxide contained in the target gas by the adsorbent in the adsorption processing, a time of the desorption processing, and a flow rate of the desorption gas are controlled on the basis of the carbon dioxide concentration of the desorption gas obtained through the concentration measurement method as recited in claim 1.

3. A carbon dioxide recovery method according to claim 2, wherein
the method further includes a purge processing of removing, by means of the residual gas, carbon dioxide present around the adsorbent from which carbon dioxide has been desorbed, and
any one or more physical properties from among a time of the purge processing and a flow rate of the residual gas employed in the purge processing are controlled on the basis of the carbon dioxide concentration of the desorption gas obtained through the concentration measurement method.

4. A carbon dioxide recovery method, the method comprising:
an adsorption processing of causing carbon dioxide contained in a target gas to be adsorbed by an adsorbent; and
a desorption processing of desorbing carbon dioxide from the adsorbent, to thereby yield a desorption gas containing carbon dioxide, wherein
the method further includes a mixing process of controlling the amount of the desorption gas added to the target gas on the basis of the carbon dioxide concentration of the desorption gas obtained through the concentration measurement method as recited in claim 1, and,
in the adsorption processing, a gas mixture formed by mixing the target gas with the desorption gas is brought into contact with the adsorbent.

5. A carbon dioxide recovery device which is adapted to cause carbon dioxide contained in a target gas to be adsorbed by an adsorbent, to desorb carbon dioxide from the adsorbent, and to yield a desorption gas containing carbon dioxide, wherein the device comprises:
a first measurement unit that can detect a flow rate and a carbon dioxide concentration of the target gas;
a second measurement unit that can detect a flow rate and a carbon dioxide concentration of a residual gas resulting from adsorption of carbon dioxide contained in the target gas by the adsorbent; and
a calculation unit that can determine a carbon dioxide concentration of the desorption gas on the basis of the measurement results obtained in the first measurement unit and the second measurement unit.

6. A recovery device according to claim 5, which further includes a control unit that can control, on the basis of the carbon dioxide concentration of the desorption gas,
any one or more physical properties from among a time of an adsorption processing of causing carbon dioxide contained in the target gas to be adsorbed by the adsorbent,
a flow rate of a residual gas resulting from adsorption of carbon dioxide contained in the target gas by the adsorbent in the adsorption processing,
a time of a desorption processing of desorbing carbon dioxide from the adsorbent, to thereby yield a desorption gas containing carbon dioxide, and a flow rate of the desorption gas.

7. A recovery device according to claim 5 or 6, wherein
the device further includes a purge unit adapted to conduct a purge processing of removing, by means of the residual gas, carbon dioxide present around the adsorbent from which carbon dioxide has been desorbed, and
the purge unit can control any one or more physical properties from among a time of the purge processing and a flow rate of the residual gas in the purge processing on the basis of the carbon dioxide concentration of the desorption gas.

8. A recovery device according to claim 5 or 6, wherein
the device further includes a mixing unit adapted to conduct a mixing process of controlling the amount of desorption gas mixed with the target gas on the basis of the carbon dioxide concentration of the desorption gas, and,
in the mixing unit, a gas mixture formed by mixing the target gas with the desorption gas is brought into contact with the adsorbent.
